# EUROPEAN PATENT SPECIFICATION

(11) **EP 3 612 392 B1**
(45) Date of publication and mention of the grant of the patent: **30.12.2020**
(21) Application number: 18725663.1
(22) Date of filing: 16.04.2018
(51) Int. Cl.: B41J 2/14, B41M 5/42

(54) **SYSTEM AND METHOD FOR LASER INDUCED FORWARD TRANSFER COMPRISING A MICROFLUIDIC CHIP PRINT HEAD WITH A RENEWABLE INTERMEDIATE LAYER**
SYSTEM UND VERFAHREN FÜR LASERINDUZIERTEN VORWÄRTSTRANSFER MIT EINEM MIKROFLUIDISCHEN CHIPDRUCKKOPF MIT EINER ERNEUERBAREN ZWISCHENSCHICHT
SYSTÈME ET PROCÉDÉ DE TRANSFERT DIRECT INDUIT PAR LASER COMPRENANT UNE TÊTE D'IMPRESSION À PUCE MICROFLUIDIQUE AYANT UNE COUCHE INTERMÉDIAIRE RENOUVELABLE

(30) Priority: 16.04.2017 US 201762485952 P
(43) Date of publication of application: 26.02.2020
(73) Proprietor: Precise Bio Inc., Winston Salem, North Carolina 27101 (US); Precise Bio 3D Ltd, 73199 Hevel Modiin (IL)
(72) Inventor: BATT, Aryeh, 90440 Beit Yatir (IL); EITAN, Amos, 93123 Jerusalem (IL); EISENBACH, Ariel, 44865 Zufim (IL)
(74) Representative: Lecomte & Partners
(86) International application number: PCT/IL2018/050429
(87) International publication number: WO 2018/193446

(56) References cited:
- JP-A- H11 105 276
- US-A- 4 531 138
- US-A1- 2009 115 820
- US-A1- 2016 296 933
- US-B1- 6 474 783

## Description

### REFERENCE TO RELATED PUBLICATIONS

This application is a European regional phase entry of International (PCT) Pat. Appl. No. PCT/IL2018/050429, filed 16 April 2018, and claims priority from U.S. Provisional Pat. Appl. No. 62/485,952, filed 16 April 2017.

### FIELD OF THE INVENTION

This invention relates in general to means and methods for Laser Induced Forward Transfer (LIFT). It relates in particular to a microfluidic head for LIFT that incorporates a renewable intermediate layer.

### BACKGROUND OF THE INVENTION

Laser Induced Forward Transfer (LIFT) is a printing process, particularly for 3-D printing, that is useful for printing of rigid, highly viscous, or sensitive materials. LIFT methods currently known in the art are of limited efficiency in some uses (particularly for bio-printing) because they tend to require a long preliminary preparation process, complex system design, and materials having a limited shelf life. A general review of the state of the art can be found in the article "Laser Direct-Write Techniques for Printing of Complex Materials" (Arnold, C.B.; Serra, P.; Piqué, A. MRS Bull. 32, 2007, 23-31).

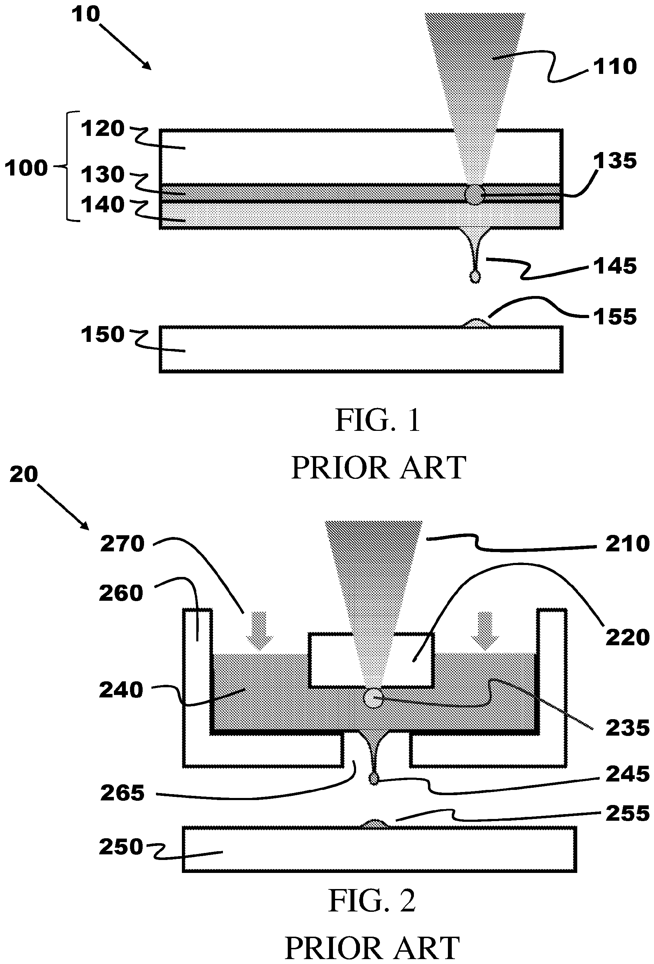

The standard LIFT process known in the art is illustrated schematically in FIG. **1****.** A typical LIFT system **10** comprises a block **100** that delivers material to a receiving substrate **150** upon activation by the output **110** of a pulsed laser (the laser itself is not shown in the figure). Block **100** is suspended in the air above the receiving substrate. Block **100** comprises a substrate **120** that is made of a material that is transparent at the wavelength of the laser output. Substrate **120** is coated with an intermediate layer **130** that is made from a material, typically a metal or polymer, that absorbs strongly at the wavelength of the laser output. A third layer **140** comprising the material to be deposited on the receiving substrate (hereinafter referred to as "ink") coats the intermediate layer, facing the receiving substrate as shown in the illustration. The laser is typically focused on the intermediate layer so that each pulse will deliver its energy to a small spot at the interface between transparent substrate **120** and the intermediate layer.

When the laser is activated, the light pulse delivers energy to a spot on the intermediate layer, creating a bubble of vapor **135.** When the bubble of vapor collapses, it generates a transient high pressure in the direction of the ink layer **140,** thereby forming a jet of ink **145,** which is deposited on a region **155** of the receiving substrate. The relative positions of block **100,** laser output **110,** and receiving substrate **150** are then adjusted such that each successive laser pulse irradiate fresh intermediate layer, and deposits the ink on a different region of the receiving substrate if so desired. If the ink itself absorbs sufficiently strongly at the wavelength of the laser output, then the ink layer can be coated directly onto the transparent substrate without any need for the intermediate layer.

U.S. Pat. No. 9446618 discloses an improved LIFT system and method. In this "Renewable LIFT" system **20,** schematically illustrated in FIG. **2****,** instead of separate ink and intermediate layers, the system utilizes liquid or gel ink **240** contained in a reservoir **260** that has an orifice **265** facing the receiving substrate **250.** Inlet **270** allows a constant flow of ink into the reservoir. In Renewable LIFT, laser output **210** is focused via transparent substrate **220** directly into the ink. Vapor bubble **235** is formed within the reservoir and upon collapse produces a pressure spike that forces a jet of ink **245** through the orifice and onto the receiving substrate **250** at location **255.** Reservoir inlet **270** permits refilling of the reservoir, or alternatively, a constant flow of ink into and through the reservoir.

While Renewable LIFT allows high throughput and resolution of 10 µm or even less, it also suffers from some drawbacks. Because of mixing, diffusion, and variable flow stability, it is not possible to include an intermediate layer in Renewable LIFT systems. Thus, it is not possible to perform Renewable LIFT using inks that do not absorb strongly at the output wavelength of the laser. Moreover, precise control of the width and flow velocity of the ink layer is difficult, and can vary over time due to such factors as the humidity, atmospheric pressure, etc.

Thus, there remains a long-felt yet unmet need for a LIFT method and system that can overcome these limitations of currently known LIFT systems while maintaining high throughput and high resolution, and that comprises relatively inexpensive components. Other LIFT printing systems are known from US 4 531 138 A and US 6 474 783 B1.

### SUMMARY OF THE INVENTION

The system disclosed herein is designed to meet this long-felt need. Instead of an arrangement that includes components such as block **100** or reservoir **260**, the inventive system and method uses an integrated microfluidic chip (MFC) that controls and stabilizes the flow of ink. The MFC comprises three regions: an upper region comprising material that is substantially transparent at the laser output wavelength; a lower region comprising a small orifice through which droplets of ink are ejected in the direction of a receiving substrate; and a middle region into which two channels are embedded, with a portion of the channels open to each other along their sides over a region opposite the orifice. A fluid that absorbs strongly at the output wavelength of the laser flows through the upper channel, and ink flows through the lower channel. Laser light impinges on the fluid flowing through the upper channel, heating it and thereby leading to the formation of a transient pressure wave that forces ink out of the orifice, similar to the process that occurs in LIFT systems known in the art. The shallowness of the channels decreases the Reynolds number of the flows, thereby maintaining the ink thickness and velocity in front of the orifice by suppressing turbulence within the flow. Due to the small Reynolds numbers and the small size of the channels, the two fluids form uniform layers with stable laminar flow, so that no significant mixing occurs over the region in which the two channels are in contact.

The scope of the invention is defined by the appended claims.

### BRIEF DESCRIPTION OF THE DRAWINGS

The invention will now be described with reference to the drawings, wherein:
FIG. **1** illustrates schematically the principles of LIFT as known in the art;
FIG. **2** illustrates schematically a second type of LIFT system known in the art; and,
FIG. **3** illustrates schematically one embodiment of a LIFT system of the present invention that incorporates a microfluidic chip with a renewable intermediate layer as a LIFT head, with FIG. **3A** showing a schematic cross-sectional view and FIG. **3B** showing a schematic three-dimensional view illustrating the flow of ink through the microfluidic chip;
FIG. **4** illustrates schematically a second embodiment of a LIFT system of the present invention;
FIG. **5** illustrates schematically a portion of a third embodiment of a LIFT system of the present invention in which ink jet ejection occurs from the narrow side of the microfluidic chip; and,
FIGS. **6A** and **6B** illustrate schematically bottom and side views of a fourth embodiment of a LIFT system of the present invention in which ink jet ejection occurs perpendicular to the direction of propagation of the laser light beam that acts as the LIFT energy source.

### DETAILED DESCRIPTION OF THE PREFERRED EMBODIMENTS

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof.

As used herein, the abbreviation "LIFT" stands for "Laser Induced Forward Transfer."

As used herein, the abbreviation "MFC" stands for "microfluidic chip."

As used herein, the abbreviation "PMMA" stands for "poly(methyl methacrylate)."

As used herein, the abbreviation "COC" "cyclic olefin copolymer."

As used herein, the abbreviation "PDMS" stands for "polydimethylsiloxane."

As used herein, the term "ink" refers to any substance that is deposited from the MFC head onto a receiving substrate.

As used herein, with reference to LIFT printing technology, the term "ink jet" refers to ink expelled from a LIFT device to be deposited on a substrate. The "jet" may comprise a stream of ink or one or more droplets.

As used herein, with reference to numerical quantities the term "about" refers to a value within a range of ± 25% of the nominal quantity.

In addition, in the following description of the geometry of the MFC print head, the terms "upper," "middle," and "lower" are used to describe three regions into which the MFC is divided. The terms do not necessarily refer to the absolute physical locations of the regions in space, but rather are used for simplicity and convenience to describe the general construction of the MFC. In this context, the term "upper" refers to that region of the MFC through which energy is transferred from an energy source to ink located in an ink channel inside the MFC; the term "lower" refers to that region of the MFC that contains an orifice from which ink is expelled from the MFC head towards a receiving substrate; and the term "middle" refers to that region of the MFC that contains a channel through which ink flows during the LIFT process. While in some embodiments of the invention, the regions are disposed generally vertically one above the other, the terms are not to be construed to limit the construction of the MFC to any specific geometric arrangement of the three regions.

In the following description of the LIFT system of the present invention, for simplicity, embodiments of the system and method are described in which the output of a pulsed laser is used as the energy source for creating the jet of ink that is sent to the receiving substrate. Since the general principle of LIFT requires only the formation of a pressure transient rather requiring that the pressure transient be caused specifically as a result of absorption of energy emanating from a laser, any method for creating an energy pulse sufficiently strong to cause the creation of an ink jet via forward transfer of energy is considered by the inventors to be within the scope of the invention. Non-limiting examples of energy sources other than pulsed lasers that can be used in the present invention include electric arcs, flashlamps, and ultrasonic resonators.

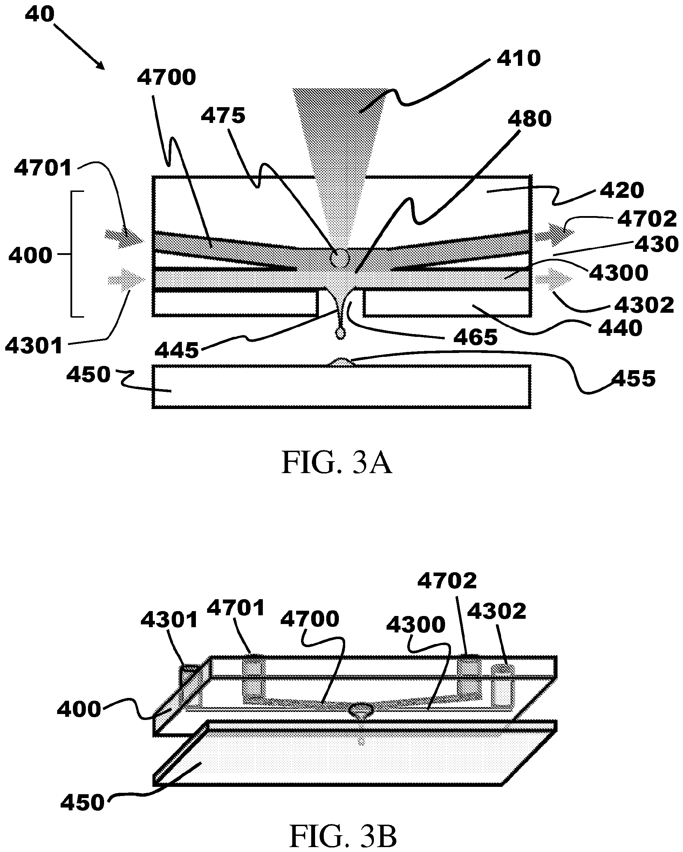

Reference is now made to FIG. **3**, which presents a schematic diagram of a typical non-limiting embodiment **40** of a LIFT system that includes a microfluidic chip LIFT head of the present invention. A cross-sectional view is shown in FIG. **3A**, while FIG. **3B** shows a view from beneath the LIFT head that illustrates the inlet and outlet connections of the fluid flow channels of the MFC LIFT head. The system comprises an MFC **400**, which serves as the LIFT head, and a receiving substrate **450** onto which ink is deposited from the LIFT head. The MFC comprises three regions: (1) a relatively thick upper region **420**; (2) a relatively thin middle region **430** comprising at least two channels that pass through the body of the MFC: an intermediate layer channel **4700** and an ink channel **4300**, which is disposed further from the upper region than is intermediate layer channel **4700**; and (3) a relatively thin lower region **440** that includes an orifice **465** facing the receiving substrate.

A fluid connection **480** between is provided between the two channels within the MFC. In preferred embodiments, the fluid connection is opposite orifice **465**, that is, the fluid connection is positioned such that a line connecting the center of orifice **465** and the area over which the energy source enters the upper region of the MFC (e.g. the center of the laser beam) will pass through the fluid connection.

In the embodiment of the MFC LIFT head illustrated schematically in FIG. **3**, the two channels are curved and are linked to each other opposite the orifice. A gap in walls of the channels in this area of linkage provides the fluid connection between them.

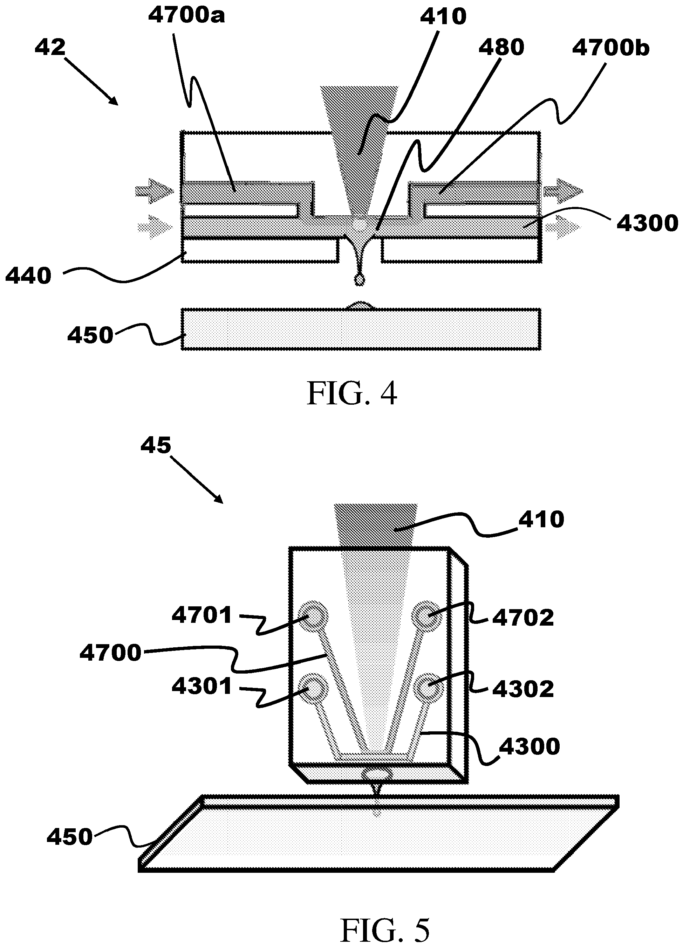

Reference is now made to FIG. **4**, which presents a schematic illustration of a second non-limiting embodiment **42** of the system of the present invention. In the embodiment shown in the figure, the intermediate layer channel is divided into two portions, **4700a** and **4700b,** the interior end of each of which connects to the ink channel within the MFC. In this embodiment, fluid connection **480** is provided by the two connections between the intermediate layer and ink channels.

The MFC can be made of any appropriate material. The following non-limiting examples of materials of construction are given to assist the person of ordinary skill in the art how to make and use the invention disclosed herein; no limitations on physical or chemical properties such as rigidity or elasticity of materials suitable for use in the MFC are to be inferred from the following discussion. Typical materials of construction include polymers such as poly(methyl methacrlyate) (PMMA), cyclic olefin copolymer (COC), and polydimethylsiloxane (PDMS), as well as materials such as glass, metal, ceramic, etc. In some embodiments of the invention, the MFC is made of a polymer such as PMMA, and glass is embedded in the upper region (described in detail below) in order to prevent damage to the chip by the high temperatures and pressures that are developed during the LIFT process. In some other embodiments of the invention, the MFC is made of a polymer such as PDMS bonded to a glass slide, according to methods that are well-known in the art.

Upper region **420** is constructed of a material that will allow the energy that drives the LIFT process to pass through to the intermediate layer channel. For example, when a laser is used as the energy source, upper region **420** is sufficiently transparent to laser output **410** to permit enough light to pass through the upper region such that light impinging on the intermediate layer retains enough energy to evaporate enough of the material in the intermediate layer to produce a vapor bubble **475** that will, upon collapse, provide a pressure transient sufficiently great to force ink out of the orifice in the direction of the receiving substrate.

Reference is now made to FIG. **5**, which presents schematically a third non-limiting embodiment **45** of the LIFT head of the instant invention. In this embodiment, the ink jet is emitted from the narrow side of the MFC rather than from the broad side as shown in FIGs. **3** and **4****.**

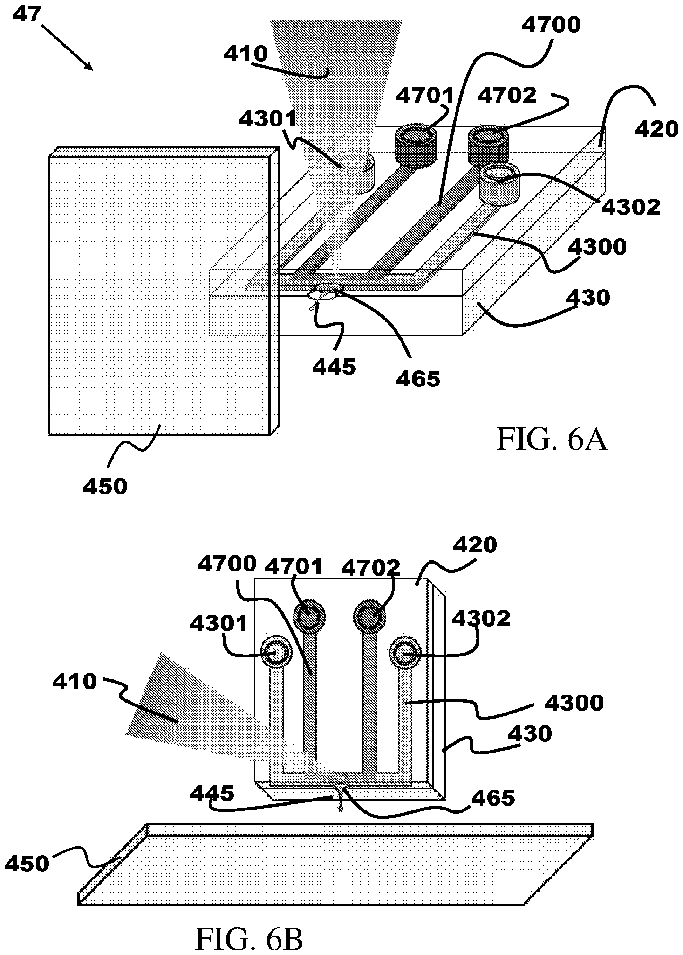

Reference is now made to FIG. **6**, which presents schematically bottom (FIG. **6A**) and side (FIG. **6B**) views of a fourth non-limiting embodiment **47** of the LIFT head of the instant invention. In the embodiment illustrated in FIG. **6**, laser output **410** is not coplanar with ink channel **4300** or intermediate layer channel **4700,** but rather is at an angle (in preferred embodiments, perpendicular) to the plane of the ink channel. In these embodiments, the three regions of the MFC are not arranged vertically one above the other. Rather, the lower region, which includes orifice **465**, is oriented to the side of the upper region through which the laser beam passes, and the middle region, which contains the ink flow and intermediate layer channels. Thus, in the embodiment shown in FIG. **6**, the "lower region" is actually more of a conceptual region than a separate physical layer. In the embodiment illustrated in the figure, the inlets and outlets of the channels are located on the same face of the MFC as that through which the laser beam passes, i.e. the ink and intermediate layer materials flow through the upper layer to reach channels **4300** and **4700.** The ink jet **445** exits from a side of the MFC that is adjacent to the side through which the laser beam enters the MFC. It is known in the art that the ink jet formed during the LIFT process does not necessarily have to propagate in the same direction as the laser beam; see, for example, "Laser-Generated Liquid Microjets: Correlation between Bubble Dynamics and Liquid Ejection" (Patrascioiu, A.; Fernández-Pradas, J. M.; Palla-Papavlu, A.; Morenza, J. L.; Serra, P. Microfluid Nanofluid 16, 2014, 55), and "Time-Resolved Imaging of the Laser Forward Transfer of Liquids" (Duocastella, M.; Fernández-Pradas, J. M.; Morenza, J. L.; Serra, P. J. Appl. Phys. 106, 2009, 084907). The inlets and outlets may, however, be located on any position of the MFC that is convenient for the specific use to which the LIFT system is being put, and for the optimal overall geometry of the system for that specific use. Alternative geometries such as those in which the inlet and outlet are located on one or more of the sides of the MFC perpendicular to the side through which the laser beam passes are considered by the inventors to be within the scope of the invention.

In the embodiments illustrated in FIGs. **3 - 6**, the MFC printing head comprises a single orifice. Embodiments of the invention in which the printing head comprises a plurality of orifices, each of which is in fluid connection with the ink channel, are considered by the inventors to be within the scope of the invention.

In preferred embodiments such as the ones illustrated in FIGs. **3 - 6**, the fluid connection between the two channels is a few mm in length.

The sizes of the channels are optimized for the particular material being used. In typical embodiments of the invention, the height of each channel is between 20 µm and 1 mm, and its width is between 50 µm and 3 mm. As a general rule, a smaller channel height will result in smaller droplets being ejected as the ink jet, and will require less energy per pulse than a higher channel would need. On the other hand, if the material flowing through the channel is highly viscous, a channel having smaller dimensions will result in a larger pressure drop, especially in the cases of highly viscous inks flowing through the MFC at a high velocity, which can lead to several possible problems, such as clogging of the ink in the channel, leaking of ink from the orifice in the bottom region due to uneven flow, and in severe cases, even breaking of the chip. In addition, flow under these conditions is difficult to generate and control by use of ordinary pumps.

In some embodiments of the invention, the two channels have different interior dimensions. In general, the height of the intermediate layer channel will depend on the details of the particular application in which the LIFT system is being used, while the total width of the two channels will define the total length scale of the LIFT process. For example, in embodiments in which the energy source is a laser, then the optimum height of the intermediate layer channel will be determined by the absorbance of the material that flows through the intermediate layer at the laser output wavelength and the laser output energy at that wavelength, with the height being determined by the optical depth necessary for sufficient energy to be absorbed to provide the vapor bubble that drives the LIFT process.

In some embodiments of the invention, the inlet and outlet of each channel have different dimensions with the ink channel varying from its one end to the other, in order to provide better pressure control at the orifices. In some embodiments of the invention, the inlet and the outlet comprise standard connectors to connect the ink and intermediate layer channels to the conduits that transfer the ink and intermediate layer material to and from the MFC LIFT head.

The LIFT system depicted in FIGs. **3 - 6** comprises a source of energy (e.g. laser output **410**). Intermediate layer channel **4700** comprises an inlet **4701** and an outlet **4702,** and ink channel **4300** comprises an inlet **4301** and an outlet **4302.** In typical embodiments of the invention, connections between channel inlets and outlets and external tubes are made by standard microfluidic connectors. The external tubes provide a fluid connection between the MFC and at least one pump that creates the flow of ink and the material of the intermediate layer through the channel. Non-limiting examples of the types of pumps that can be used with the MFC head disclosed herein include peristaltic, pressure, and syringe pumps. The required flow rate of the fluids will depend on the required refresh rate, which depends inter alia on the droplet sizes in the ink jet and the desired printing rate. In typical embodiments of the invention, the flow rate is between 1 mm s⁻¹ and 1 m s⁻¹. In some embodiments of the invention, the volumetric flow rate is less than 0.01 µL s⁻¹.

The flow rate can be governed by either volumetric flow rate control or by pressure difference control. In some embodiments of the invention, the stability of the liquid level at the orifice is maintained by having the suction at the exit be greater than the pressure at the inlet. In other non-limiting embodiments of the invention, the liquid level and shape at the orifice are monitored by a closed feedback loop; by measuring the pressure inside the MFC, at the inlet, or at the outlet; or by measuring the shape of the meniscus formed by the ink at the orifice.

Other non-limiting optional components of the LIFT system disclosed herein include an air trap, pressure sensors, pressure regulators, flow-rate sensors, valves, and temperature control apparatus.

The orifice can be of any shape or size appropriate to the desired output of the LIFT system. As non-limiting examples, shape and size of the orifice can be chosen for purposes such as controlling the LIFT process; stabilizing the flow through the MFC; controlling the shape of the meniscus at the orifice; and preventing unwanted ejection of liquid from or introduction of air bubbles into the MFC. In typical embodiments of the invention, the orifice will have either a round or a square shape. In some non-limiting embodiments of the invention, the orifice may have either a trapezoidal or an elliptical shape. In some other non-limiting embodiments of the invention, the orifice has non-uniform boundaries. The diameter of the orifice typically ranges from 100 µm to 1 mm, depending on the viscosity and flow rate of the ink.

In some embodiments of the invention, the orifice is coated or subject to or other surface treatment that will alter the contact angle of the ink droplets being formed. The coating or surface treatment may be of any appropriate type known in the art, and prepared by any appropriate method known in the art.

In typical embodiments of the LIFT system, such as those shown in FIGs. **3 - 6**, the LIFT head faces a substrate **450.** The LIFT process is then use to deposit ink **455** on the substrate, as described in detail below.

It is within the scope of the invention to disclose a LIFT method that is based on the use of an MFC LIFT head. In a typical embodiment of the LIFT method disclosed herein, ink flows through ink channel **4300**, entering the MFC through inlet **4301** and exiting through outlet **4302.** Simultaneously, intermediate layer material, which strongly absorbs the input energy that drives through the process, flows through intermediate layer channel **4700**, entering the MFC through inlet **4701** and exiting through outlet **4702.** As described above, fluid connection **480** between the two channels enables contact of the two materials; downstream of the fluid connection, the two channels separate in order to enable recycling of the intermediate layer material and of the ink. Because the design of the MFC LIFT head ensures laminar flow of the intermediate material and of the ink, there will be essentially no mixing of the two materials even though there is a the fluid connection between the channels that carry the materials.

Laser output **410** is focused on the intermediate layer channel, preferably in the region of the channel where the fluid connection between the intermediate layer channel and the ink channel is found. The laser light is at least partially absorbed by the material in the intermediate layer channel, thereby causing a transient pressure wave. Without being bound by theory, there are several plausible mechanisms by which the transient pressure wave can be created. For example, if the intermediate layer material absorbs sufficient energy for it to at least partially evaporate, a vapor bubble will be formed that, upon collapse, will cause a transient pressure increase. In another possible mechanism for creation of the transient pressure wave, the absorption of light by the intermediate layer material may be sufficient to heat the intermediate layer material without evaporating it, thereby causing a transient pressure wave as the material expands. In a third possible mechanism, the laser light ablates or partially ablates intermediate layer material, thereby causing the transient pressure wave. Which mechanism or combination of mechanisms is operative in a particular system will depend on details of the system such as the nature of the intermediate layer material, the wavelength and pulse energy of the laser output, and so on.

The pressure wave caused by the absorption of energy in the intermediate layer passes to the ink flowing through the ink layer via the fluid connection connecting the intermediate layer and ink layer channels, causing ink to be forced through orifice **465** toward the receiving substrate **450,** thereby creating ink jet **445** that is then deposited (**455**) on the receiving substrate. In contrast to normal LIFT processes, however, in the instant invention, fresh sample is brought before the laser by the aforementioned flow of intermediate layer material and ink through the MFC rather than by movement of the LIFT head or laser.

The channels in the MFC may be prepared by any method known in the art. In preferred embodiments of the invention, the channels in the MFC are prepared by ablating, molding, mechanical milling, or embossing the thick layer in the desired shape. The lower region is then used to seal the channels.

The wavelength, polarization, and mode distribution of the laser light are restricted only by the requirement that the absorption coefficient for absorption of the laser output by the intermediate layer must be sufficiently high that a single laser pulse is sufficient to at least partially evaporate the ink that absorbs the light in order to create the vapor bubble and eventually the ink jet that exits the MFC via the orifice. In preferred embodiments of the invention, the absorption coefficient of the intermediate layer material at the laser wavelength is at least 1000 cm⁻¹.

The system and method of the present invention are thus particularly useful in cases in which the ink itself does not absorb strongly at the laser output wavelength.

For the printing of living cells, a liquid or gel medium is used that has mechanical, biological, and optical properties appropriate to the specific application for which the system is being used. Commercially available bio-inks can be used in the invention herein disclosed. Non-limiting examples of materials from which compatible bio-inks can be made include collagen, gelatin, alginate, gellan gum, polyethylene glycol, and hyaluronic acid. Most of these bio-inks are hydrogels comprising an aqueous solution of polymer and cross-linker. These bio-inks tend to have dynamic viscosities (µ) between 200 and 10⁴ mPa•s at low shear rates. At higher flow rates, these gels tend to show shear thinning behavior having an approximately power-law relation with flow behavior index n = 0 (i.e. µ = Kτ⁻¹, where τ is the shear rate and K is a constant). The viscosity may be selected to have a value that is appropriate to the particular application or specific biological tissue to which the LIFT printing is being applied.

In preferred embodiments of the invention, the laser pulse duration is ≤ 10 ns. The use of lasers that produce picosecond or femtosecond pulses are within the scope of the invention. In preferred embodiments of the invention, the laser fluence at the focus is greater than 0.2 J cm⁻².

In typical embodiments of the invention, the laser light travels through free space, directed as needed by beam steering and focusing elements known in the art such as galvanometers, mirrors, simple lenses, and microscope objectives. In typical embodiments, the laser output is focused to a spot size having a radius of between 1 µm and 1 mm. The spot size will depend on the particular application, as the size of the droplets of ink exiting the head is directly related to the laser spot size.

While embodiments of the invention in which the laser light travels to the MFC via free space are considered by the inventors to be within the scope of the invention, in some preferred embodiments of the invention, the laser energy is transferred to the ink via an optical fiber. Because of the high laser pulse energies used in the LIFT process, in preferred embodiments of the invention in which optical fibers are used to transfer the laser energy to the LIFT head, hollow-core optical fibers or photonic-crystal fibers (PCFs) are used. In some embodiments of the invention, the fiber is inserted into the MFC and immersed in the ink. In some preferred embodiments of the invention in which optical fibers are used to transfer the laser energy to the LIFT head and in which the optical fiber is kept outside of the MFC, collimation of the light at the end of the fiber is done by embedding a focuser at the end of the fiber. Any appropriate type of focuser known in the art can be used. In some embodiments of the invention a scanner or other alignment means is used in addition to the focusing elements.

In some embodiments of the invention, the system comprises a plurality of MFC LIFT printing heads. These embodiments are particularly useful for cases in which different inks are to be applied to the substrate or if a high printing rate is desired. In cases in which inks of different materials are used, the different MFC heads can be designed with different dimensions or architectures optimized for the properties of the ink. As a non-limiting example, the diameters of the ink channels can be set to optimize the flow for materials of different viscosities.

In the embodiments in which multiple printing heads are used, separate lasers can be used for each printing head, or a single laser can be used and its output divided among the heads either physically, for example by using beamsplitters or a plurality of optical fibers, or temporally by alternating the head to which the laser light is sent. As a non-limiting example, in a system with 5 printing heads for which a 10 kHz printing rate is desired, a laser firing at a 50 kHz repetition rate can be used with successive pulses directed to successive heads, or a single laser firing at a 10 kHz repetition rate in which the output is divided physically among the printing heads can be used instead. In some embodiments of the invention in which multiple printing heads are used, the system includes a single reservoir for ink or a single reservoir for intermediate layer material or both. All of the printing heads are connected, either in series or in parallel, to the single reservoir.

The embodiment of the invention illustrated schematically in FIG. **3** shows the MFC LIFT head located above the receiving substrate. Because gravitational effects are not very significant on the length scale of the LIFT process disclosed herein, the MFC LIFT head may be placed in any convenient orientation relative to the receiving substrate, and all possible orientations of the MFC LIFT head relative to the receiving substrate, including but not limited to cases in which the ink jet is expelled horizontally or in which the MFC LIFT head is located below the receiving substrate are considered by the inventors to be within the scope of the invention.

LIFT systems that incorporate the MFC LIFT head described above are also within the scope of the invention. As a non-limiting example of how the LIFT head and receiving substrate may be positioned and oriented, in some embodiments of LIFT systems that incorporate an MFC LIFT head, the position and orientation of at least one of the MFC or the receiving substrate are controlled by placing the component on an XYZ stage. As another non-limiting embodiment of how the LIFT head and receiving substrate may be positioned and oriented, in some other embodiments of LIFT systems that incorporate an MFC LIFT head, at least one of the MFC or the receiving substrate is placed on an XY stage, and the other component is placed on stage or other mount that allows it to move in the Z direction (i.e. controls the distance between that component and the component mounted on the XY stage). The stages or mounts upon which the components are placed are preferably electrically actuated and preferably having a precision of ±1 µm or better in each direction. In other embodiments The MFC LIFT head is typically placed so that the orifice is between 50 µm and a few mm from the receiving substrate. One of ordinary skill in the art will appreciate that the exact distance between the orifice and the receiving substrate will depend on parameters of the specific application, for example, the specific properties of the ink used and the droplet size and shape required by the application.

In some embodiments of the invention, the LIFT system additionally includes a source of light placed so as to irradiate the receiving substrate and thereby stimulate the secondary gelation or curing of the ink after it has been deposited on the receiving substrate. The light may be of any wavelength suitable to effect activation of the hydrogel, and is typically in the visible or UV range, depending primarily on the cross-linker used. The irradiation of the receiving substrate may be performed continuously during printing, at the end of the process, or, particularly in 3D printing applications, after each layer of ink has been deposited on the receiving substrate.

The upper limit on the printing rate is determined primarily by the laser repetition rate, the maximum flow rate of the ink, and the surface re-initialization time. Printing rates of 1 MHz or more are possible in principle with the LIFT system disclosed herein. In practice, the upper limit on the printing rate is generally set by the viscosity of the ink. LIFT systems that incorporate the MFC head disclosed herein typically have printing rates of -10 kHz.

The LIFT system disclosed herein is not limited to applications for which high repetition rates are necessary or desirable. Repetition rates of below 100 Hz are quite feasible with the LIFT system, such as bio-printing applications that have typical repetition rates of on the order of 1 Hz are quite feasible with the LIFT system disclosed herein. In fact, the LIFT system disclosed herein is capable of printing even a single drop of ink.

Particularly for biological applications such as printing of biological solutions and the use of cell-based bio-inks, the system additionally includes apparatus for controlling the environment, preferably apparatus that can control at least the temperature, humidity, and CO₂ and O₂ levels. Typical printing temperatures for biological applications are between 4 °C and 37 °C. Any appropriate type of environmental control system known in the art may be used. In addition, in biological applications, the system is preferably placed in a sterile hood that comprises air-flow control, and in preferred embodiments of the system as used in biological applications, the printing head is sterilized prior to use and most preferably during use as well as needed to prevent contamination.

## Claims

1. A Laser-Induced Forward Transfer (LIFT) printing system, comprising:
an energy source;
a receiving substrate (450); and, at least one printing head (400) disposed between said
energy source and said receiving substrate; the LIFT printing system is configured to create an ink jet by a pressure transient within said printing head following delivery of a pulse of energy from said energy source to said printing head and cause the ink jet exit said printing head toward said receiving substrate, said printing head comprising a microfluidic chip (MFC), said MFC comprising three regions: an upper region (420);
a middle region (430) comprising:
at least one ink channel (4300) passing through said MFC, said at least one ink channel comprising an ink channel inlet (4301) and an ink channel outlet (4302);
at least one intermediate layer channel (4700) passing through said MFC, said at least one intermediate layer channel comprising an intermediate layer channel inlet (4701) and an intermediate layer channel outlet (4702); and,
a fluid connection (480) within said MFC between said at least one ink channel and said at least intermediate layer channel; and,
a lower region (440), said lower region comprising an orifice (465) in fluid connection with said ink channel;
said at least one ink channel and said at least one intermediate layer channel being disposed at said fluid connection (480) such that said ink channel lies between said intermediate layer channel and said orifice;
an ink reservoir in fluid connection with said ink channel inlet; and,
an intermediate layer material reservoir in fluid connection with said intermediate layer channel inlet.

2. The LIFT printing system according to claim 1, wherein said energy source comprises at least one laser providing pulsed output (410).

3. The LIFT printing system according to claim 2, comprising at least one of the following: focusing means configured to focus said laser output to a spot **characterized by** a radius of between about 1 µm and about 1 mm; and, at least one optical fiber disposed so as to transmit said pulsed output from said laser to said printing head.

4. The LIFT printing system according to claim 2, comprising intermediate layer material, wherein said laser is configured to provide output **characterized by** at least one of the following:
said output is at a wavelength at which said intermediate layer material absorbs and of sufficient energy such that upon irradiation by said output, said intermediate layer material is at least partially evaporated, ablated, or thermally expanded; and,
said output is at a wavelength at which said intermediate layer material absorbs and of sufficient energy such that upon irradiation by said output, said intermediate layer material absorbs sufficient energy such that a transient pressure wave is created within said intermediate layer material.

5. The LIFT printing system according to claim 1, comprising at least one of: pumping means for pumping ink from said ink reservoir to said ink channel inlet and through said ink channel; pumping means for pumping intermediate layer material from said intermediate layer reservoir to said intermediate layer channel inlet and through said intermediate layer channel; pumping means for recirculating ink from said ink reservoir through said ink channel inlet and back to said ink reservoir; and, pumping means for recirculating intermediate layer material from said intermediate layer reservoir through said intermediate layer channel and back to said intermediate material reservoir.

6. The LIFT printing system according to claim 1, wherein one of the following is true: at least one of said head and said receiving substrate is mounted on an XYZ stage having a precision of ±1 µm or better in each direction; and, one of said head and said receiving substrate is mounted on an XY stage having a precision of ± 1 µm or better in each direction, and the other component is mounted on a stage or mount configured to move said other component along a Z axis.

7. The LIFT printing system according to claim 1, comprising n printing heads, n > 1.

8. The LIFT printing system according to claim 7, wherein said energy source is configured to irradiate each of said n printing heads sequentially; and said energy source comprises preferably *n* energy sources, each of which is configured to irradiate one of said n printing heads.

9. A method for Laser-Induced Forward Transfer (LIFT) printing, wherein said method comprises:
obtaining a LIFT printing system comprising:
an energy source;
a receiving substrate (450); and,
at least one printing head (400) disposed between said energy source and said receiving substrate such that an ink jet created by a pressure transient within said printing head following delivery of a pulse of energy from said energy source to said printing head will exit said printing head toward said receiving substrate, said printing head comprises a microfluidic chip (MFC), said MFC comprising three regions: an upper region (420);
a middle region (430) comprising:
at least one ink channel (4300) passing through said MFC, said at least one ink channel comprising an ink channel inlet (4301) and an ink channel outlet (4302);
at least one intermediate layer channel (4700) passing through said MFC, said at least one intermediate layer channel comprising an intermediate layer channel inlet (4701) and an intermediate layer channel outlet (4702); and,
a fluid connection (480) within said MFC between said at least one ink channel and said at least intermediate layer channel; and, a lower region (440), said lower region comprising an orifice (465) in fluid connection with said ink channel;
said at least one ink channel and said at least one intermediate layer channel being disposed at said fluid connection (480) such that said ink channel lies between said intermediate layer channel and said orifice;
an ink reservoir in fluid connection with said ink channel inlet; and, an intermediate layer material reservoir in fluid connection with said intermediate layer channel inlet;
flowing ink from said ink reservoir through said at least one ink channel (4300);
flowing intermediate layer material from said intermediate layer reservoir through said at least one intermediate layer channel (4700);
irradiating said intermediate layer material with output (410) of an energy source, said output having sufficient energy at a location at which said intermediate layer material is irradiated to at least partially evaporate said intermediate layer material at said fluid connection (480), such that upon collapse of a vapor bubble formed upon said irradiation, a transient pressure increase is created, thereby forcing ink out of said orifice; and,
receiving said ink forced out of said orifice on at least one predetermined location (455) on a receiving substrate (450).

10. The method according to claim 9, wherein said step of irradiating comprises irradiating with output of a pulsed laser at a wavelength at which said intermediate material absorbs.

11. The method according to claim 9, wherein said step of irradiating comprises transmitting said pulsed output from said laser to said printing head by means of an optical fiber.

12. The method according to claim 9, wherein said step of flowing ink from said ink reservoir through said at least one ink channel comprises recirculating said ink back into said ink reservoir.

13. The method according to claim 9, wherein said step of flowing intermediate layer material from said intermediate layer material reservoir through said at least one intermediate layer channel comprises recirculating said intermediate layer material back into said intermediate layer material reservoir.

14. The method according to claim 9, wherein said step of flowing comprises flowing said ink through channels in n printing heads, n > 1, and said step of irradiating comprises irradiating ink in each of said n printing heads.

15. The method according to claim 9, comprising moving said printing head relative to said substrate during said LIFT printing process.

## Patentansprüche

1. Laser-induziertes Vorwärtstransfer (LIFT)-Drucksystem
umfassend:
eine Energiequelle;
ein Aufnahmesubstrat (450) und mindestens einen Druckkopf (400), der zwischen der Energiequelle und dem Empfangssubstrat angeordnet ist, wobei das LIFT-Drucksystem konfiguriert ist einen Tintenstrahl durch einen Druckübergang innerhalb des Druckkopfs nach Abgabe eines Energieimpulses von der Energiequelle an den Druckkopf zu erzeugen und zu bewirken, dass der Tintenstrahl den Druckkopf in Richtung des Empfangssubstrats verlässt, wobei der Druckkopf einen Mikrofluidik-Chip (MFC) umfasst, wobei der MFC drei Bereiche umfasst:
einen oberen Bereich (420);
einen mittleren Bereich (430), umfassend:
mindestens einen Tintenkanal (4300), der durch den MFC verläuft, wobei der mindestens eine Tintenkanal einen Tintenkanaleinlass (4301) und einen Tintenkanalauslass (4302) umfasst;
mindestens einen Zwischenschichtkanal (4700), der durch den MFC verläuft, wobei der mindestens eine Zwischenschichtkanal einen Zwischenschichtkanaleinlass (4701) und einen Zwischenschichtkanalauslass (4702) umfasst; und
eine Fluidverbindung (480) innerhalb des MFC zwischen dem mindestens einen Tintenkanal und dem mindestens Zwischenschichtkanal; und
einen unteren Bereich (440), wobei der untere Bereich eine Öffnung (465) in Fluidverbindung mit dem Tintenkanal umfasst;
wobei der mindestens eine Tintenkanal und der mindestens eine Zwischenschichtkanal an der Fluidverbindung (480) so angeordnet sind, dass der Tintenkanal zwischen dem Zwischenschichtkanal und der Öffnung liegt;
ein Tintenreservoir in Fluidverbindung mit dem Tintenkanaleinlass;
ein Zwischenschichtmaterialreservoir in Fluidverbindung mit dem Zwischenschichtkanaleinlass.

2. LIFT-Drucksystem nach Anspruch 1, wobei die Energiequelle mindestens einen Laser umfasst, der eine gepulste Ausgabe liefert (410).

3. LIFT-Drucksystem nach Anspruch 2, umfassend mindestens eines der folgenden: Fokussiermittel, die konfiguriert sind, die Laserleistung auf einen Punkt zu fokussieren, der durch einen Radius zwischen ungefähr 1 µm und ungefähr 1 mm gekennzeichnet ist, und mindestens eine optische Faser derart angeordnet, um die gepulste Ausgabe von dem Laser an den Druckkopf zu übertragen.

4. LIFT-Drucksystem nach Anspruch 2, umfassend Zwischenschichtmaterial, wobei der Laser konfiguriert ist, eine Leistung bereitzustellen, die durch mindestens eines der folgenden Merkmale gekennzeichnet ist:
die Leistung liegt bei einer Wellenlänge, bei der das Zwischenschichtmaterial absorbiert, und von ausreichender Energie, so dass das Zwischenschichtmaterial bei Bestrahlung durch die Leistung zumindest teilweise verdampft, abgetragen oder thermisch expandiert wird; und
diese Leistung bei einer Wellenlänge liegt, bei der das Zwischenschichtmaterial absorbiert wird, und von ausreichender Energie ist, so dass bei Bestrahlung durch diese Leistung, das Zwischenschichtmaterial ausreichend Energie aufnimmt, so dass innerhalb des Zwischenschichtmaterials eine transiente Druckwelle entsteht.

5. LIFT-Drucksystem nach Anspruch 1, umfassend eines der folgenden:
Pumpmittel zum Pumpen von Tinte von dem Tintenreservoir zum Tintenkanaleinlass und durch den Tintenkanal;
Pumpmittel zum Pumpen von Zwischenschichtmaterial von dem Zwischenschichtreservoir zu dem Zwischenschichtkanaleinlass und durch den Zwischenschichtkanal;
Pumpmittel zum Umwälzen von Tinte von dem Tintenreservoir durch den Tintenkanaleinlass und zurück zu dem Tintenreservoir; und
und Pumpmittel zum Umwälzen von Zwischenschichtmaterial von dem Zwischenschichtreservoir durch den Zwischenschichtkanal und zurück zu dem Zwischenmaterialreservoir.

6. LIFT-Drucksystem nach Anspruch 1, wobei eines der folgenden wahr ist:
mindestens einer dieser Köpfe und das Aufnahmesubstrat sind auf einer XYZ-Stufe mit einer Genauigkeit von 1 µm oder besser in jeder Richtung angebracht; und
einer dieser Köpfe und dieses Aufnahmesubstrats sind auf einer XY-Stufe mit einer Genauigkeit von 1 µm oder besser in jeder Richtung montiert, und das andere Bauteil ist auf einer Stufe oder Halterung montiert, die konfiguriert ist, dieses andere Bauteil entlang einer Z-Achse zu bewegen.

7. LIFT-Drucksystem nach Anspruch 1, umfassend n Druckköpfe, n > 1.

8. LIFT-Drucksystem nach Anspruch 7, wobei die Energiequelle konfiguriert ist, jeden der n Druckköpfe nacheinander zu bestrahlen; und
die Energiequelle vorzugsweise n Energiequellen umfasst, die konfiguriert sind, einen der n Druccköpfe zu bestrahlen.

9. Verfahren für einen Laser-induzierten Vorwärtstransfer (LIFT)-Druck, wobei das Verfahren umfasst:
Erhalt eines LIFT-Drucksystems, umfassend:
eine Energiequelle
ein Aufnahmesubstrat (450); und
mindestens einen Druckkopf (400), der zwischen der Energiequelle und dem Aufnahmesubstrat angeordnet ist, dergestalt, dass ein Tintenstrahl, der durch einen Druckübergang in dem Drucckopf nach Abgabe eines Energieimpulses von der Energiequelle an den Druckkopf erzeugt wird, den Druckkopf in Richtung des Aufnahmesubstrats verlässt, wobei der Druckkopf einen Mikrofluidik-Chip (MFC) umfasst, wobei der MFC drei Bereiche umfasst:
einen oberen Bereich (420);
einen mittleren Bereich (430), umfassend:
mindestens einen Tintenkanal (4300), der durch die MFC verläuft, wobei der mindestens eine Tintenkanal einen Tintenkanaleinlass (4301) und einen Tintenkanalauslass (4302) umfasst; mindestens einen Zwischenschichtkanal (4700), der durch den MFC verläuft, wobei der mindestens eine Zwischenschichtkanal einen Zwischenschichtkanaleinlass (4701) und einen Zwischenschichtkanalauslass (4702) umfasst; und
eine Fluidverbindung (480) innerhalb des MFC zwischen dem mindestens einen Tintenkanal und dem mindestens Zwischenschichtkanal und einen unteren Bereich (440), wobei der untere Bereich eine Öffnung (465) in Fluidverbindung mit dem Tintenkanal umfasst;
wobei der mindestens eine Tintenkanal und der mindestens eine Zwischenschichtkanal an der Fluidverbindung (480) so angeordnet sind, dass der Tintenkanal zwischen dem Zwischenschichtkanal und der Öffnung liegt;
ein Tintenreservoir in Fluidverbindung mit dem Tintenkanaleinlass; und ein Zwischenschichtmaterialreservoir in Fluidverbindung mit dem Zwischenschichtkanaleinlass;
wobei Tinte aus dem Tintenreservoir durch den mindestens einen Tintenkanal (4300) fließt;
wobei Zwischenschichtmaterial aus dem Zwischenschichtreservoir durch den mindestens einen Zwischenschichtkanal (4700) fließt;
wobei das Zwischenschichtmaterial mit einer Leistung (410) einer Energiequelle bestrahlt wird, wobei die Leistung an einer Stelle, an der das Zwischenschichtmaterial bestrahlt wird, ausreichend Energie hat, um das Zwischenschichtmaterial an dem Fluidanschluss (480) zumindest teilweise zu verdampfen, so dass beim Kollaps einer bei der Bestrahlung gebildeten Dampfblase ein vorübergehender Druckanstieg erzeugt wird, wodurch Tinte aus der Öffnung herausgedrückt wird; wobei die aus der Öffnung herausgedrückte Tinte an mindestens einer vorbestimmten Stelle (455) auf einem Aufnahmesubstrat (450) aufgenommen wird.

10. Verfahren nach Anspruch 9, wobei der Schritt der Bestrahlung eine Bestrahlung mit der Leistung eines gepulsten Lasers bei einer Wellenlänge umfasst, bei das Zwischenmaterial absorbiert.

11. Verfahren nach Anspruch 9, wobei der Schritt der Bestrahlung die Übertragung der gepulsten Leistung von dem Laser zu dem Druckkopf mittels einer optischen Faser umfasst.

12. Verfahren nach Anspruch 9, wobei der Schritt des Fließens der Tinte aus dem Tintenreservoir durch den mindestens einen Tintenkanal die Rückführung der Tinte zurück in das Tintenreservoir umfasst.

13. Verfahren nach Anspruch 9, wobei der Schritt des Fließens von Zwischenschichtmaterial aus dem Zwischenschichtmaterialreservoir durch den mindestens einen Zwischenschichtkanal die Rückführung des Zwischenschichtmaterials in das Zwischenschichtmaterialreservoir umfasst.

14. Verfahren nach Anspruch 9, wobei der Schritt des Fließens das Fließen der Tinte durch Kanäle in n Druckköpfen n> 1 umfasst und der Bestrahlungsschritt das Bestrahlen von Tinte in jedem der n Druckköpfe umfasst.

15. Verfahren nach Anspruch 9, umfassend das Bewegen des Druckkopfes relativ zu dem Substrat während des LIFT-Druckprozesses.

## Revendications

1. Système d'impression du type à transfert-avant induit par laser (LIFT) qui comprend :
une source d'énergie ;
un substrat de réception (450) ; et
au moins une tête d'impression (400) qui est disposée entre ladite source d'énergie et ledit substrat de réception ;
le système d'impression de type LIFT étant configuré pour créer un jet d'encre par l'intermédiaire d'un transitoire de pression au sein de ladite tête d'impression suite à la distribution d'une impulsion d'énergie à partir de ladite source d'énergie dans la direction de ladite tête d'impression et qui fait en sorte que le jet d'encre quitte ladite tête d'impression dans la direction dudit substrat de réception, ladite tête d'impression comprenant un laboratoire sur puce (MFC), ledit MFC comprenant trois zones :
une zone supérieure (420) ;
une zone médiane (430) qui comprend :
au moins un canal pour l'encre (4300) qui traverse ledit (MFC), ledit au moins un canal pour l'encre comprenant une entrée de canal pour l'encre (4301) et une sortie de canal pour l'encre (4302) ;
au moins un canal de couche intermédiaire (4700) qui traverse ledit MFC, ledit au moins un canal de couche intermédiaire comprenant une entrée de canal de couche intermédiaire (4701) et une sortie de canal de couche intermédiaire (4702) ; et
une connexion par fluide (480) au sein dudit MFC entre ledit au moins un canal pour l'encre et ledit au moins un canal de couche intermédiaire ; et
une zone inférieure (440), ladite zone inférieure comprenant un orifice (465) qui est mis en connexion par fluide avec ledit canal pour l'encre ;
ledit au moins un canal pour l'encre et ledit au moins un canal de couche intermédiaire étant disposés à l'endroit occupé par ladite connexion par fluide (480) d'une manière telle que ledit canal pour l'encre est situé entre ledit canal de couche intermédiaire et ledit orifice ;
un réservoir pour l'encre mis en connexion par fluide avec ladite entrée de canal pour l'encre ; et
un réservoir de matière de couche intermédiaire mis en connexion par fluide avec ladite entrée de canal de couche intermédiaire.

2. Système d'impression de type LIFT selon la revendication 1, dans lequel ladite source d'énergie comprend au moins un laser qui procure une sortie pulsée (410).

3. Système d'impression de type LIFT selon la revendication 2, qui comprend au moins un élément choisi parmi ceux indiqués ci-après : un moyen de focalisation qui est configuré pour focaliser ladite sortie laser sur un point qui est **caractérisé par** un rayon qui se situe entre environ 1 µm et environ 1 mm ; et au moins une fibre optique qui est disposée de manière à transmettre ladite sortie pulsée depuis ledit laser jusqu'à ladite tête d'impression.

4. Système d'impression de type LIFT selon la revendication 2, qui comprend une matière de couche intermédiaire ; dans lequel ledit laser est configuré pour procurer une sortie qui est **caractérisée par** au moins un des éléments repris ci-après :
ladite sortie est réglée à une longueur d'onde à laquelle ladite matière de couche intermédiaire absorbe et possède une énergie suffisante pour que, suite à une exposition aux rayonnements par ladite sortie, ladite matière de couche intermédiaire est soumise à une évaporation, à une ablation ou à une dilatation thermique au moins partielle ; et
ladite sortie est réglée à une longueur d'onde à laquelle ladite matière de couche intermédiaire absorbe et possède une énergie suffisante pour que, suite à une exposition aux rayonnements par ladite sortie, ladite matière de couche intermédiaire absorbe une énergie suffisante pour créer une onde de pression transitoire au sein de ladite matière de couche intermédiaire.

5. Système d'impression de type LIFT selon la revendication 1, qui comprend au moins un élément choisi parmi ceux indiqués ci-après :
un moyen de pompage qui est destiné à pomper de l'encre partir dudit réservoir pour l'encre dans la direction de ladite entrée du canal pour l'encre et à travers ledit canal pour l'encre ;
un moyen de pompage qui est destiné à pomper une matière de couche intermédiaire à partir dudit réservoir de couche intermédiaire dans la direction de ladite entrée du canal de couche intermédiaire et à travers ledit canal de couche intermédiaire ;
un moyen de pompage qui est destiné à remettre en circulation de l'encre qui émane dudit réservoir pour l'encre à travers ladite entrée de canal pour l'encre et en retour dans la direction dudit réservoir pour l'encre ; et
un moyen de pompage qui est destiné à remettre en circulation de la matière de couche intermédiaire à partir dudit réservoir de couche intermédiaire à travers ledit canal de couche intermédiaire et en retour dans la direction dudit réservoir de matière intermédiaire.

6. Système d'impression de type LIFT selon la revendication 1, dans lequel une des affirmations suivantes est vraie :
au moins un élément choisi parmi ladite tête et ledit substrat de réception est monté sur un étage XYZ qui possède une précision de ± 1 µm ou mieux dans chaque direction ; et
au moins un élément choisi parmi ladite tête et ledit substrat de réception est monté sur un étage XY qui possède une précision de ± 1 µm ou mieux dans chaque direction, et l'autre composant est monté sur un étage ou sur un support qui est configuré pour déplacer ledit autre composant le long d'un axe des Z.

7. Système d'impression de type LIFT selon la revendication 1, qui comprend n têtes d'impression, dans lequel n > 1.

8. Système d'impression de type LIFT selon la revendication 7, dans lequel ladite source d'énergie est configurée pour exposer chacune desdites n têtes d'impression à des rayonnements d'une manière séquentielle ; et ladite source d'énergie comprend de préférence n sources d'énergie, chacune étant configurée pour exposer une desdites n têtes d'impression à des rayonnements.

9. Procédé destiné à une impression du type à transfert-avant induit par laser (LIFT) ; dans lequel ledit procédé comprend le fait de :
obtenir un système d'impression de type LIFT qui comprend :
une source d'énergie ;
un substrat de réception (450) ; et
au moins une tête d'impression (400) qui est disposée entre ladite source d'énergie et ledit substrat de réception d'une manière telle qu'un jet d'encre créé par un transitoire de pression au sein de ladite tête d'impression, suite à la distribution d'une impulsion d'énergie à partir de la source d'énergie dans la direction de ladite tête d'impression, va quitter ladite tête d'impression dans la direction dudit substrat de réception, ladite tête d'impression comprenant un laboratoire sur puce (MFC), ledit MFC comprenant trois zones :
une zone supérieure (420) ;
une zone médiane (430) qui comprend :
au moins un canal pour l'encre (4300) qui traverse ledit (MFC), ledit au moins un canal pour l'encre comprenant une entrée de canal pour l'encre (4301) et une sortie de canal pour l'encre (4302) ;
au moins un canal de couche intermédiaire (4700) qui traverse ledit MFC, ledit au moins un canal de couche intermédiaire comprenant une entrée de canal de couche intermédiaire (4701) et une sortie de canal de couche intermédiaire (4702) ; et
une connexion par fluide (480) au sein dudit MFC entre ledit au moins un canal pour l'encre et ledit au moins un canal de couche intermédiaire ; et
une zone inférieure (440), ladite zone inférieure comprenant un orifice (465) qui est mis en connexion par fluide avec ledit canal pour l'encre ;
ledit au moins un canal pour l'encre et ledit au moins un canal de couche intermédiaire étant disposés à l'endroit occupé par ladite connexion par fluide (480) d'une manière telle que ledit canal pour l'encre est situé entre ledit canal de couche intermédiaire et ledit orifice ;
un réservoir pour l'encre mis en connexion par fluide avec ladite entrée de canal pour l'encre ; et
un réservoir de matière de couche intermédiaire mis en connexion par fluide avec ladite entrée de canal de couche intermédiaire ;
faire en sorte que de l'encre s'écoule à partir dudit réservoir pour l'encre en passant par ledit au moins un canal pour l'encre (4300) ;
faire en sorte qu'une matière de couche intermédiaire s'écoule à partir dudit réservoir de couche intermédiaire à travers ledit au moins un canal de couche intermédiaire (4700) ;
exposer à des rayonnements ladite matière de couche intermédiaire avec une sortie (410) d'une source d'énergie, ladite sortie possédant une énergie suffisante à un endroit auquel ladite matière de couche intermédiaire est exposée à des rayonnements dans le but d'évaporer au moins en partie ladite matière de couche intermédiaire à l'endroit occupé par ladite connexion par fluide (480), d'une manière telle qu'après la rupture d'une bulle de vapeur que l'on obtient suite à ladite exposition à des rayonnements, on obtient une augmentation de la pression transitoire, ce qui donne lieu au fait que de l'encre quitte de manière forcée ledit orifice ; et
recevoir ladite encre qui a quitté ledit orifice de manière forcée à au moins un endroit prédéterminé (455) sur un substrat de réception (450).

10. Procédé selon la revendication 9, dans lequel ladite étape d'exposition à des rayonnements comprend une exposition à des rayonnements avec une sortie d'un laser pulsé à une longueur d'onde dans laquelle ladite matière intermédiaire absorbe.

11. Procédé selon la revendication 9, dans lequel ladite étape d'exposition à des rayonnements comprend la transmission de ladite sortie pulsée à partir dudit laser jusqu'à ladite tête d'impression au moyen d'une fibre optique.

12. Procédé selon la revendication 9, dans lequel ladite étape dans laquelle on fait circuler de l'encre à partir dudit réservoir pour l'encre en passant par ledit au moins un canal pour l'encre comprend la remise en circulation de ladite encre en retour dans la direction dudit réservoir pour l'encre.

13. Procédé selon la revendication 9, dans lequel ladite étape dans laquelle on fait circuler de la matière de couche intermédiaire à partir dudit réservoir de matière de couche intermédiaire en passant par ledit au moins un canal de couche intermédiaire comprend la remise en circulation de ladite matière de couche intermédiaire en retour dans la direction dudit réservoir de matière de couche intermédiaire.

14. Procédé selon la revendication 9, dans lequel ladite étape dans laquelle on fait circuler ladite encre à travers des canaux dans n têtes d'impression, n > 1, et ladite étape dans laquelle on procède à exposition à des rayonnements comprennent le fait d'exposer de l'encre à des expositions dans chacune desdites n têtes d'impression.

15. Procédé selon la revendication 9, qui comprend le fait de déplacer ladite tête d'impression par rapport au substrat en question au cours dudit procédé d'impression de type LIFT.
